Europäisches Patentamt

European Patent Office

Office européen des brevets

(19)

(11) Publication number: **0 134 076**
**B1**

# (12) EUROPEAN PATENT SPECIFICATION

(45) Date of publication of patent specification: **26.10.88**

(51) Int. Cl.⁴: **B 01 J 29/28, C 07 C 6/12**

(21) Application number: **84303815.9**

(22) Date of filing: **06.06.84**

(54) A process for preparing a coked zeolite catalyst and a process for the selective disproportionation of toluene using said catalyst.

(30) Priority: **19.08.83 US 524626**
**19.08.83 US 524722**

(43) Date of publication of application:
**13.03.85 Bulletin 85/11**

(45) Publication of the grant of the patent:
**26.10.88 Bulletin 88/43**

(84) Designated Contracting States:
**BE DE FR GB IT NL**

(56) References cited:
**EP-A-0 021 600**
**DE-A-2 714 239**
**US-A-3 525 775**
**US-A-4 231 899**

(73) Proprietor: **MOBIL OIL CORPORATION**
**150 East 42nd Street**
**New York New York 10017 (US)**

(72) Inventor: **Chang, Elaine Jaqueline**
**31 Spring Hill Road**
**Mantua, NJ 08051 (US)**
Inventor: **Graziani, Kenneth Richard**
**Apt. X-1, Kinswick Apartments**
**Thorofare, NJ 08086 (US)**

(74) Representative: **Cooper, John Anthony et al**
**Mobil Court 3 Clements Inn**
**London WC2A 2EB (GB)**

Courier Press, Leamington Spa, England.

## Description

This invention relates to a process for preparing a coked zeolite catalyst and a process for the selective disproportionation of toluene using said catalyst.

It is known from US—A—4,097,543 that toluene can be selectively disproportionated to para-xylene utilizing a zeolite catalyst which has been treated to deposit a controlled amount of coke thereon. It has, however, now been discovered that a substantial reduction in the liquid hourly space velocity at which the zeolite is contacted with the coke generating material in US—A—4,097,543 substantially improves the subsequent performance of the coked catalyst.

Of the xylene isomers, i.e., ortho-, meta- and para-xylene, ortho and para-xylene are the more desired products. Para-xylene is of particular value in the manufacture of terephthalic acid which is an intermediate in the manufacture of synthetic fibers. Mixtures of xylene isomers, either alone or in further admixture with ethylbenzene, generally containing a concentration of 24 weight percent para-xylene in the equilibrium mixture have previously been separated by expensive superfractionation and multi-stage refrigeration steps. As will be apparent, such processes have involved high operating costs and resulted in limited yields.

In one aspect the present invention resides in a process for preparing a coke-containing catalyst which process comprises contacting a crystalline zeolite having a silica to alumina ratio of at least 12 and a constraint index within the range of 1 to 12, with a thermally decomposable organic compound at a temperature in excess of the decomposition temperature of said compound but less than 649°C (1200°F) at a hydrogen to organic compound mole ratio of between 0 and 1 and is characterised by a controlled liquid hourly space velocity of between 0.5 and 3.5 to deposit at least 2 weight percent coke thereon.

Preferably, the organic compound is contacted with the catalyst at a liquid hourly space velocity of 2. The coke selectivated zeolite catalyst preferably has a xylene sorption capacity greater than 1 gram/per 100 grams of zeolite and an ortho xylene sorption time for 30% of said capacity of greater than 100 minutes, the sorption capacity and sorption time being measured at 120°C and a xylene pressure of 0.6±0.1 kPa (4.5±0.8 mm of mercury).

In a further aspect, the present invention resides in a process for the selective production of para-xylene by disproportionation of toluene in the presence of a catalyst comprising a crystalline zeolite having a silica to alumina ratio of at least 12 and a constraint index within the range of 1 to 12, which catalyst has undergone controlled precoking by exposing the same to a thermally decomposable organic compound at a temperature in excess of the decomposition temperature of said compound but less than 649°C (1200°F), at a hydrogen to organic compound mole ratio of between 0 and 1 and a liquid hourly space velocity of between 0.5 and 3.5 to deposit at least 2 weight percent coke thereon and thereafter contacting toluene with the resulting coke-containing catalyst under disproportionation conditions including a temperature between 371°C and 552°C (700°F and 1025°F) and a hydrogen to toluene mole ratio greater than 1 and up to 10 and recovering a product mixture containing para-xylene in an amount greater than the thermodynamic equilibrium concentration thereof in the total xylenes produced. In a preferred embodiment, toluene is employed as the source of coke for deposition on the surface of the zeolite catalyst, and is brought into initial contact with the catalyst at a temperature from 427°C to 649°C (800°F to 1200°F). Thereafter the toluene feed is disproportionated in the presence of the coke-containing catalyst under the conditions indicated above.

Organic materials, thermally decomposable under the above temperature conditions to provide coke deposition, encompass a wide variety of compounds including by way of example, hydrocarbons, such as paraffinic, cycloparaffinic, olefinic, cyclolefinic and aromatic; oxygen-containing organic compounds such as alcohols, aldehydes, ethers, ketones and phenols; heterocyclics such as furans, thiophenes, pyrroles and pyridines. Usually it is contemplated that a thermally decomposable hydrocarbon, such as an alkyl-substituted aromatic, will be the source of coke and most preferably toluene itself. In the latter case, toluene is initially brought into contact under conditions of temperature and hydrogen concentration amenable to rapid coke formation and when the desired coke deposition has been effected, toluene feed is continued in contact with the coke-containing catalyst under conditions of temperature and hydrogen concentration conducive to disproportionation, with a greatly reduced coking rate.

Where the thermally decomposable organic material is toluene or a compound of similar reactivity, the decomposition temperature employed is preferably greater than 538°C (1000°F). With organic compounds that are more readily decomposable than toluene such as, for example, phenols and styrene, precoking can be effectively carried out at temperatures less than 538°C (1000°F). With the use of higher temperatures within the aforenoted range, the presence of hydrogen has been found to be unnecessary. With temperatures of less than 593°C (1100°F) preferably some hydrogen, generally at least 0.2 mole of hydrogen per mole of organic compounds is desirable to yield a more stable catalyst after the catalyst has been coked. Contact between toluene feed and the pre-coked catalyst is then established under disproportionation conditions including a temperature between 371°C and 552°C (700°F and 1000°F), preferably between 399°C and 510°C (750°F and 950°F) and a hydrogen to toluene mole ratio greater than 1 and up to 10, preferably between 1.2 and 10. Preferably, the disproportionation is conducted at a pressure between 101 kPa and 10100 kPa (1 atmosphere and 100 atmospheres) and a weight hourly space velocity between 0.5 and 50.

The amount of coke deposited on the catalyst, prior to contact with toluene under disproportionation conditions, is at least 2 weight percent, but preferably should not exceed 60 weight percent. The optimum amount of coke to be employed in a given toluene disproportionation process will depend on the conditions at which the desired disproportionation is effected including among other variables, the crystal size of the aluminosilicate zeolite used and the nature of the catalyst binder, if any, employed.

While it is contemplated that the process described herein may involve use of a crystalline zeolite of any crystal size, it is preferred that the zeolite predominantly (i.e. greater than 50 percent by weight) consist of crystals having a size greater than 0.5 micron, more preferably in the approximate range of 1 to 20 microns and particularly 1 to 6 microns. It has been found that as a general rule, the smaller the zeolite crystal size, the greater the amount of coke deposition required to achieve comparable results. Thus, on a binder-free basis, it has been observed that with the use of small zeolite crystals, e.g., in the range of 0.02 to 0.05 micron size, greater than 20 percent of coke deposition is required to obtain results comparable to those obtained with larger zeolite crystals, e.g., in the range of 1 to 2 microns, having approximately 4 weight percent of coke deposited thereon. In assessment of zeolite crystal size, conventional scanning electron microscopy (SEM) techniques can be used, the minimum crystal dimension of a given crystal being taken as the dimension of reference.

The amount and nature of the binder composited with the crystalline zeolite also has been found to have a marked effect on the amount of coke deposition required to obtain the desired selective production of para-xylene. Thus, while binder-free zeolite of 1 to 2 micron size afforded high para-xylene selectivity with 4 weight percent of coke deposited thereon, comparable use of a composite of such zeolite (65 percent) and alumina (35 percent) required an average of 22 weight percent of coke deposition. With the use of small zeolite crystals (0.02 to 0.05 micron) and alumina binder (35 percent), it is contemplated that 40 percent or more coke deposition would be required to obtain the desired high para-xylene selectivity. Also, increase in alumina content of the zeolite composite, which desirably is in the form of an extrudate, would be expected to require increased coke deposition to obtain comparable high para-xylene selectivity. With the use of other binders, such as clay or silica, it is anticipated that the amount of coke required for comparable results may be somewhat less than in the case where alumina is the sole binding material.

The precoke crystalline zeolites employed in the process described herein are further desirably characterized by certain hydrocarbon sorption capacities and rates. Measurements of such properties are conveniently carried out gravimetrically in thermal balance. In particular, it has been found that an equilibrium sorption capacity of xylene, which can be either para, meta, ortho or a mixture thereof, preferably para-xylene since this isomer reaches equilibrium within the shortest time of at least 1 gram per 100 grams of zeolite measure at 120°C and a xylene pressure of 0.6±0.1 kPa (4.5±0.8 mm of mercury) and an ortho-xylene sorption time for 30 percent of said capacity of greater than 100 minutes (at the same conditions of temperature and pressure) are required in order to achieve the desired selective production of para-xylene.

It has been found that zeolites exhibiting very high selectivity for para-xylene production require a very long time up to and exceeding a thousand minutes to sorb o-xylene in an amount of 30% total xylene sorption capacity. For those materials it is more convenient to determine the sorption time for a lower extent of sorption, such as 5%, 10% or 20% of capacity, and to estimate the 30% sorption time by applying the following multiplication factors F as illustrated for 5% sorption:

| $t_{0.3}=F \cdot t_{0.05}$ Percent of sorption capacity | Factor (F) to Estimate 30% Sorption Time |
|---|---|
| 5 | 36 |
| 10 | 9 |
| 20 | 2.2 |

The term "zeolite" is used generally herein to define natural or synthetic porous tectosilicates characterized by having a rigid crystalline framework structure composed of an assembly of silicon atoms and at least a trace amount of a trivalent metal atom, for example iron, boron, gallium, chromium and the like or mixtures thereof, but preferably aluminum, the silicon atoms and trivalent metal atoms each being surrounded by a tetrahedron of shared oxygen atoms, and a precisely defined pore structure.

The crystalline zeolites utilized herein are members of a class of zeolitic materials which exhibit unusual properties. Although these zeolites have unusually low alumina contents, i.e. high silica to alumina mole ratios, they are very active even when the silica to alumina mole ratio exceeds 30. The activity is surprising since catalytic activity is generally attributed to framework aluminum atoms and/or cations associated with these aluminum atoms. These zeolites retain their crystallinity for long periods in spite of the presence of steam at high temperature which induces irreversible collapse of the framework of other zeolites, e.g. of the X and A type.

An important characteristic of the crystal structure of this novel class of zeolites is that it provides a selective constrained access to and egress from the intracrystalline free space by virtue of having an effective pore size intermediate between the small pore Linde A and the large pore Linde X, i.e., the pore windows of the structure are of a size such as would be provided by 10-membered rings of silicon atoms interconnected by oxygen atoms. It is to be understood, of course, that these rings are those formed by the regular disposition of the tetrahedra making up the anionic framework of the crystalline zeolite, the oxygen atoms themselves being bonded to the silicon (or aluminum, etc.) atoms at the centers of the tetrahedra.

The silica to alumina mole ratio referred to may be determined by conventional analysis. This ratio is meant to represent, as closely as possible, the ratio in the rigid anionic framework of the zeolite crystal and to exclude aluminum in the binder or in cationic or other form within the channels. Although zeolites with silica to alumina mole ratios of at least 12 are useful, it is preferred in some instances to use zeolites having substantially higher silica-alumina ratios, e.g., 1600 and above. In addition, zeolites as otherwise characterized herein but which are substantially free of aluminum, that is zeolites having silica to alumina mole ratios of up to infinity, are found to be useful and even preferable in some instances. Such "high silica" or "highly siliceous" zeolites are intended to be included within this description. Also included within this definition are substantially pure silica analogs of the useful zeolites described herein, that is to say those zeolites having no measurable amount of aluminum (silica to alumina mole ratio of infinity) but which otherwise embody the characteristics disclosed.

This class of zeolites, after activation, acquire an intracrystalline sorption capacity for normal hexane which is greater than that for water, i.e., they exhibit "hydrophobic" properties. This hydrophobic character can be used to advantage in some applications.

The novel class of zeolites useful herein have an effective pore size such as to freely sorb normal hexane. In addition, the structure must provide constrained access to larger molecules. It is sometimes possible to judge from a known crystal structure whether such constrained access exists. For example, if the only pore windows in a crystal are formed by 8-membered rings of silicon and aluminum atoms, then access by molecules of larger cross-section than normal hexane is excluded and the zeolite is not of the desired type. Windows of 10-membered rings are preferred, although in some instances excessive puckering of the rings or pore blockage may render these zeolites ineffective.

Although 12-membered rings in theory would not offer sufficient constraint to produce advantageous conversions, it is noted that the puckered 12-ring structure of TMA offretite does show some constrained access. Other 12 ring structures may exist which may be operative for other reasons and, therefore, it is not the present intention to entirely judge the usefulness of a particular zeolite solely from theoretical structural considerations.

Rather than attempt to judge from crystal structure whether or not a zeolite possesses the necessary constrained access to molecules of larger cross-section than normal paraffins, a simple determination of the constraint index may be used. The meaning of constraint index and its method of determination are described in, for example, US—A—3,905,915. The zeolites used herein have a constraint index of 1 to 12. The preferred class of zeolites used herein are ZSM-5, ZSM-11, ZSM-12, ZSM-23, ZSM-35, ZSM-38, ZSM-48, mixture thereof and other similar materials. ZSM-5 is particularly preferred.

ZSM-5 is described in US—A—3,702,886 and US—E—29,948. ZSM-11 is described in US—A—3,709,979. ZSM-12 is described in US—A—3,832,449. ZSM-23 is described in US—A—4,076,842. ZSM-35 is described in US—A—4,016,245. ZSM-38 is more particularly described in US—A—4,046,859. ZSM-48 is described in US—A—4,375,573 and US—A—4,397,827.

The specific zeolites described, when prepared in the presence of organic cations, are substantially catalytically inactive, possibly because the intra-crystalline free space is occupied by organic cations from the forming solution. They may be activated by heating in an inert atmosphere at 540°C for one hour, for example, followed by base exchange with ammonium salts followed by calcination at 540°C in air. The presence of organic cations in the forming solution may not be absolutely essential to the formation of this type zeolite; however, the presence of these cations does appear to favor the formation of this special class of zeolite. More generally, it is desirable to activate this type of catalyst by base exchange with ammonium salts followed by calcination in air at about 540°C for from 15 minutes to 24 hours.

Natural zeolites may sometimes be converted to zeolite structures of the class herein identified by various activation procedures and other treatments such as base exchange, steaming, alumina extraction and calcination, alone or in combination. Natural minerals which may be so treated include ferrierite, brewsterite, stilbite, dachiardite, epistilbite, heulandite, and clinoptilolite.

In a preferred aspect of this invention, the zeolites are selected to have a crystal framework density, in the dry hydrogen form, of not less than 1.6 grams per cubic centimeter. The dry crystal density for known structures may be calculated from the number of silicon plus aluminum atoms per 1000 cubic Angstroms, as described on Page 19 of the article "Zeolite Structure" by W. M. Meier, included in "Proceedings of the Conference on Molecular Sieves" (London, April 1967); published by the Society of chemical Industry, London, 1968.

When the crystal structure is unknown, the crystal framework density may be determined by classical pycnometer techniques. For example, it may be determined by immersing the dry hydrogen form of the zeolite in an organic solvent which is not sorbed by the crystal. Or, the crystal density may be determined by mercury porosimetry, since mercury will fill the interstices between crystals but will not penetrate the intracrystalline free space.

When synthesized in the alkali metal form, the zeolite is conveniently converted to the hydrogen form, generally by intermediate formation of the ammonium form as a result of ammonium ion exchange and calcination of the ammonium form to yield the hydrogen form. In addition to the hydrogen form, other forms of the zeolite wherein the original alkali metal has been reduced to less than 1.5 percent by weight may be used. Thus the original alkali metal of the zeolite may be replaced by ion exchange with other suitable metal cations of Groups I through VIII of the Periodic Table, including, by way of example, nickel, copper, zinc, palladium, calcium or rare earth metals.

In practicing a particularly desired chemical conversion process, it may be useful to incorporate the above-described crystalline zeolite with a matrix comprising another material resistant to the temperature and other conditions employed in the process. Such matrix material is useful as a binder and imparts greater resistance to the catalyst for the severe temperature, pressure and reactant feed stream velocity conditions encountered in many cracking processes.

Useful matrix materials include both synthetic and naturally occurring substances, as well as inorganic materials such as clay, silica and/or metal oxides. The latter may be either naturally occurring or in the form of gelatinous precipitates or gels including mixtures of silica and metal oxides. Naturally occurring clays which can be composited with the zeolite include those of the montmorillonite and kaolin families, which families include the sub-bentonites and the kaolins commonly known as Dixie, McNamee-Georgia and Florida clays or others in which the main mineral constituent is halloysite, kaolinite, dickite, nacrite or anauxite. Such clays can be used in the raw state as originally mined or initially subjected to calcination, acid treatment or chemical modification.

In addition to the foregoing materials, the zeolites employed herein may be composited with a porous matrix material, such as alumina, silica-alumina, silica-magnesia, silica-zirconia, silica-thoria, silica-beryllia, and silica-titania, as well as ternary compositions, such as silica-alumina-thoria, silica-alumina-zirconia, silica-alumina-magnesia and silica-magnesia-zirconia. The matrix may be in the form of a cogel. The relative proportions of zeolite component and organic oxide gel matrix, on an anhydrous basis, may vary widely with the zeolite content ranging from between 1 to 99 percent by weight and more usually in the range of 5 to 80 percent by weight of the dry composite.

The disproportionation process described herein can be carried out as a batch-type, semi-continuous or continuous operation utilizing a fixed or moving bed catalyst system. The catalyst after use is regenerated whereby coke is burned to a desired extent from the catalyst in an oxygen containing atmosphere, e.g., air, at an elevated temperature. Elevated pressure is also desirable. The following Examples serve to illustrate the process of this invention.

Example 1

Alumina and HZSM-5 in a ratio by weight of 65 parts of alumina to 35 parts of zeolite were combined with water to make a mixture containing 50% solids. The mixture was mulled for 15 minutes and was then extruded through a 1.6 mm (1/16 inch) die plate and dried at 121°C (250°F). The resulting extrudates after being calcined at 538° (1000°F) in a nitrogen atmosphere, were ammonium exchanged using a 1 M aqueous solution of ammonium nitrate, and washed with water. This procedure was repeated. The material was then dried at 121°C (250°F) and subsequently calcined in air for three hours at 538°C (1000°F).

Example 2

Thirty-six grams of the catalyst prepared in Example 1 were precoked by contacting with toluene at 593°C (1100°F), 3546 kPa (100 psig), a hydrogen to toluene ratio of 0.75 and a weight hourly space velocity of 2 for a period of 55 hours. Conditions were then changed to those effective for toluene disproportionation, namely 438°C (820°F), 3546 kPa (500 psig), a hydrogen to hydrocarbon ratio of 1.5 and a WHSV of 2. The product consisted of 69.9 weight percent of toluene (equivalent to 30% toluene conversion), 13.7% benzene and 13.6% mixed xylenes. The xylenes contained 93.4% of the para isomer. At the end of a 180-day study, the top and bottom portions of the catalyst contained 28.7 grams and 30.7 grams respectively of coke per 100 grams of coke-free catalyst respectively.

Example 3

Thirty-six grams of catalyst of Example 1 were precoked with toluene at 593°C (1100°F), 790 kPa (100 psig), a hydrogen to hydrocarbon ratio of 0.75 and a WHSV of 3 for a period of 44 hours. Conditions were changed to those for toluene disproportionation, namely 454°C (850°F), 3546 kPa (500 psig), a hydrogen to hydrocarbon ratio of 1.5 and a WHSV of 3. The product consisted of 71.31 weight percent toluene (28.69% toluene conversion), 12.28% benzene and 14.48% mixed xylenes. The xylene contained 84.8% of the para isomer. At the end of a one-month run, the catalyst contained 30 grams of coke per 100 grams of coke free catalyst.

In both Examples 2 and 3 the catalyst maintained its selectivity for producing para-xylene throughout the run.

Claims

1. A process for preparing a coke-containing catalyst, which process comprises contacting a crystalline zeolite having a silica to alumina ratio of at least 12 and a constraint index within the range of 1 to 12, with a thermally decomposable organic compound at a temperature in excess of the decomposition temperature of said compound but less than 649°C (1200°F) at a hydrogen to organic compound mole ratio of between 0 and 1 and is characterised by a controlled weight hourly space velocity of between 0.5 and 3.5 to deposit at least 2 weight percent coke thereon.

2. A process for the selective production of para-xylene by disproportionation of toluene, which comprises contacting toluene with the coke-containing catalyst resulting from the process of claim 1 under disproportionation conditions including a temperature between 371°C and 552°C (700°F and 1025°F) and a hydrogen to toluene mole ratio greater than 1 and up to 10 and recovering a product mixture containing para-xylene in an amount greater than the thermodynamic equilibrium concentration thereof in the total xylenes produced.

3. The process of claim 1 or claim 2 wherein said organic compound is a hydrocarbon.

4. The process of claim 1 or claim 2 wherein said organic compound is toluene.

5. The process of claim 1 or claim 2 wherein said precoking is effected at a temperature greater than 538°C (1000°F).

6. The process of claim 1 or claim 2 wherein said crystalline zeolite has a crystal size greater than 0.5 microns.

7. The process of claim 1 or claim 2 wherein said crystalline zeolite is ZSM-5.

8. The process of claim 1 or claim 2 wherein said crystalline zeolite is present in combination with a binder therefore.

9. The process of claim 1 or claim 2 wherein the crystalline zeolite is present in combination with an alumina binder.

10. The process of claim 1 or claim 2 wherein the amount of coke is between 2 and 60 weight percent.

11. The process of claim 2 wherein said disproportionation conditions include a hydrogen to toluene mole ratio between 1.2 and 10, a temperature of 399°C to 510°C (750°F to 950°F), a pressure of 101 kPa to 10100 kPa and a WHSV of 0.5 to 50.

## Patentansprüche

1. Verfahren zur Herstellung eines kokshaltigen Katalysators, wobei dieses Verfahren den Kontakt eines kristallinen Zeoliths mit einem Siliziumdioxid/Aluminiumoxid-Verhältnis von mindestens 12 und einem Zwangsindex im Bereich von 1 bis 12 mit einer thermisch zersetzbaren organischen Verbindung bei einer Temperatur oberhalb der Zersetzungstemperatur dieser Verbindung jedoch kleiner als 649°C (1200°F) bei einem Molverhältnis von Wasserstoff zur organischen Verbindung zwischen 0 und 1 umfaßt, daß durch eine geregelte stündliche Gewichts-Raum-Geschwindigkeit von zwischen 0,5 und 3,5 gekennzeichnet ist, um zumindest 2 Gew.-% Koks darauf abzulagern.

2. Verfahren zur selektiven Herstellung von Paraxylol durch Disproportionierung von Toluol, welches den Kontakt von Toluol mit dem aus dem Verfahren nach Anspruch 1 resultierenden kokshaltigen Katalysator bei Disproportionierungsbedingungen, die eine Temperatur zwischen 371°C und 552°C (700°F und 1025°F) und ein Molverhältnis von Wasserstoff zu Toluol von größer als 1 und bis zu 10 umfassen, und die Gewinnung einer Produktmischung umfaßt, die Paraxylol in einer Menge enthält, die größer also dessen thermodynamische Gleichgewichtskonzentration in den gesamten hergestellten Xylolen ist.

3. Verfahren nach Anspruch 1 oder 2, worin die organische Verbindung ein Kohlenwasserstoff ist.

4. Verfahren nach Anspruch 1 oder 2, worin die organische Verbindung Toluol ist.

5. Verfahren nach Anspruch 1 oder 2, worin die Vorverkokung bei einer Temperatur von größer als 538°C (1000°F) durchgeführt wird.

6. Verfahren nach Anspruch 1 oder 2, worin der kristalline Zeolith eine Kristallgröße von größer als 0,5 Mikrometer aufweist.

7. Verfahren nach Anspruch 1 oder 2, worin der kristalline Zeolith ZSM-5 ist.

8. Verfahren nach Anspruch 1 oder 2, worin der kristalline Zeolith in Kombination mit einem Bindemittel für diesen vorhanden ist.

9. Verfahren nach Anspruch 1 oder 2, worin der kristalline Zeolith in Kombination mit einem Aluminiumoxidbindemittel vorhanden ist.

10. Verfahren nach Anspruch 1 oder 2, worin die Menge des Koks zwischen 2 und 60 Gew.-% beträgt.

11. Verfahren nach Anspruch 2, worin die Disproportionierungsbedingungen ein Molverhältnis von Wasserstoff zu Toluol zwischen 1,2 und 10, eine Temperatur von 399°C bis 510°C (750°F bis 950°F), einen Druck von 101 kPa bis 10100 kPa und eine WHSV von 0,5 bis 50 einschließen.

## Revendications

1. Un procédé de préparation d'un catalyseur contenant du coke, ce procédé consistant à mettre une zéolite cristalline dont le rapport silice/alumine est au moins égal à 12 et l'indice de contrainte est comprise entre 1 et 12, au contact d'une matière organique décomposable à la chaleur à une température supérieure à la température de décomposition de ce dérivé mais inférieure à 649°C (1 200°F), avec un rapport molaire l'hydrogène/dérivé organique compris entre 0 et 1, ce procédé étant caractérisé par une vitesse spatiale horaire pondérale contrôlée comprise entre 0,5 et 3,5 pour déposer au moins 2% en poids de coke sur ce catalyseur.

2. Un procédé de préparation sélective de para-xylène par disproportionnement de toluène, ce procédé consistant à metre du toluène au contact du catalyseur contenant du coke obtenu dans le procédé selon la revendication 1, dans des conditions de disproportionnement incluant une température comprise entre 371°C et 552°C (700°F et 1 025°F) et un rapport molaire hydrogène/toluène supérieur à 1 et pouvant atteindre jusqu'à 10 et à récupérer un mélange de produit contenant du para-xylène en quantité supérieure à sa concentration à l'équilibre thermodynamique dans l'ensemble des xylènes obtenus.

3. Le procédé selon la revendication 1 ou la revendication 2, dans lequel cette matière organique est une hydrocarbure.

4. Le procédé selon la revendication 1 ou la revendication 2, dans lequel ce dérivé organique est le toluène.

5. Le procédé selon la revendication 1 ou la revendication 2, dans lequel ce précokage est effectué à une température supérieure à 538°C (1 000°F).

6. Le procédé selon la revendication 1 ou la revendication 2, dans lequel cette zéolite cristalline a une dimension de cristaux supérieure à 0,5 micron.

7. Le procédé selon la revendication 1 ou la revendication 2, dans lequel cette zéolite cristalline est la ZSM-5.

8. Le procédé selon la revendication 1 ou la revendication 2, dans lequel cette zéolite cristalline est présente en association à un liant de cette zéolite.

9. Le procédé selon la revendication 1 ou la revendication 2, dans lequel la zéolite cristalline est associée à un liant à base d'alumine.

10. Le procédé selon la revendication 1 ou la revendication 2, dans lequel la quantité de coke est comprise entre 2 et 60% en poids.

11. Le procédé selon la revendication 1 ou la revendication 2, dans lequel ces conditions de disproportionnement incluent un rapport molaire hydrogène/toluène comprise entre 1, 2 et 10, une température comprise entre 399°C et 510°C (750°F et 950°F), une pression comprise entre 101 et 10 100 kPa et une VHSP comprise entre 0,5 et 50.